(19) **Europäisches Patentamt / European Patent Office / Office européen des brevets**

(11) **EP 2 634 277 B1**

(12) **EUROPEAN PATENT SPECIFICATION**

(45) Date of publication and mention
of the grant of the patent:
**08.10.2014 Bulletin 2014/41**

(51) Int Cl.:
**C22C 19/07** (2006.01)  **A61L 31/00** (2006.01)
**A61L 31/02** (2006.01)  **C22F 1/10** (2006.01)

(21) Application number: **13154968.5**

(22) Date of filing: **12.02.2013**

(54) **Co-based alloy for living body and stent**

Co-basierte Legierung für lebenden Körper und Stent

Alliage à base de co pour corps vivant et endoprothèse

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.02.2012 JP 2012044546**

(43) Date of publication of application:
**04.09.2013 Bulletin 2013/36**

(73) Proprietors:
• **Seiko Instruments Inc.**
**Chiba-shi, Chiba (JP)**
• **Tohoku University**
**Aoba-ku, Sendai-shi**
**Miyagi (JP)**

(72) Inventors:
• **CHIBA, Akihiko**
**Sendai-shi,, Miyagi (JP)**

• **KUROSU, Shingo**
**Sendai-shi,, Miyagi (JP)**
• **AKASAKA, Yasunori**
**Chiba-shi,, Chiba (JP)**
• **KOBAYASHI, Tomoo**
**Chiba-shi,, Chiba (JP)**
• **SUGAWARA, Ryo**
**Chiba-shi,, Chiba (JP)**

(74) Representative: **Miller Sturt Kenyon**
**9 John Street**
**London WC1N 2ES (GB)**

(56) References cited:
**EP-A1- 2 330 227    EP-A1- 2 551 363
US-A- 4 606 887**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

FIELD OF THE INVENTION

The present invention relates to a Co-based alloy for a living body and a stent using the same. More specifically, the present invention relates to a Co-based alloy for a living body that is suitable for being provided for use in medical instruments such as instruments implanted into the living body and instruments contacting the living body, and relates to a stent using the alloy.

BACKGROUND ART

A high degree of corrosion resistance and biocompatibility are required in alloys used for medical instruments implanted into the living body and medical instruments used by being brought into direct contact with the surface of the living body. In addition, for an alloy for a stent which is used for securing blood flow by dilating a portion where angiostenosis has been caused in the living body, a high degree of strength and elastic modulus are required. The alloys are also required to be highly visible in X-rays, that is, to have a high density, to have a high degree of blood compatibility, and the like.

As the Co-based alloy for a living body that satisfies the above requirements, an alloy (ASTM standards F90: hereinafter, abbreviated to an "ASTM F90 alloy") containing Co-20Cr-15W-10Ni as main components, an alloy (see JP-A-2007-162121) composed of Co: 30% to 60%, Ni: 4% to 20%, Cr: 13% to 25%, C: 0.3% or less, Si: 2.0% or less, and Mn: 2.0% or less in terms of % by mass, and the like are known in the related art. Moreover, as the stent, a stent (see JP-T-2007-517536) which has a tubular body including an alloy obtained by adding at least one kind among Zr, Ta, and Mo to 20% or more of Ti, and specifies the yield strength, magnetic susceptibility, and mass-absorption coefficient of the alloy is known.

WO 2011/118615 discloses a Co-Cr-W system alloy for biomedical applications comprising another alloy element, which has biocompatibility and is effective in increasing stacking fault energy of the alloy. The alloy element is selected from a group consisting of Nb, Ta and Fe. The alloy contains Cr: 5% by mass to 30% by mass and W: 5% by mass to 20% by mass. The alloy may be used for a stent.

SUMMARY OF THE INVENTION

Among other biomaterials based on metals, the ASTM F90 alloy is particularly excellent in the above characteristics, and promising not only as an aortic stent, but also as a material for stents for microvessels such as a coronary stent and a stent for bile ducts in the future.

However, the ASTM F90 alloy and the alloy disclosed in JP-A-2002-130808 contain a large amount of Ni, which causes a Ni allergy, so a material for a stent that is excellent in various characteristics described above without containing Ni is required. Ni is contained to secure plastic workability, and recognized as an additive element necessary for imparting characteristics of a high degree of plastic workability for tube working which is necessary for fabricating a stent.

Therefore, if the alloy composed as above is made into a Ni-free alloy, this leads to a problem that working characteristics such as plastic workability deteriorate remarkably.

In addition, when the stent is inserted into the body, the position of the stent needs to be checked under radioscopy, so a material having high X-ray visibility is desired. However, since the stent is inserted into blood vessels, and a tubular stent for intravascular insertion and the like is formed to be extremely thin, the alloy composition in the related art is not necessarily sufficient in terms of X-ray visibility under the current circumstances, so higher X-ray visibility is required.

The present invention has been made to solve the above problems, and an object thereof is to provide a Ni-free Co-based alloy for a living body that has a high strength, a high elastic modulus, and excellent plastic workability. Another object of the present invention is to provide a Co-based alloy for a living body that has excellent X-ray visibility. In addition, still another object of the present invention is to provide a stent using the alloy.

The present invention has the following constitution as means for solving the above problems.

A first aspect of the present invention is a Co-based alloy for a living body as defined in claim 1.

A second aspect of the present invention is a stent as defined in claim 4.

For the Co-based alloy for a living body of the present invention, to an alloy based on Co-Cr-W, Fe which exhibits biocompatibility and has an effect of increasing a stacking fault energy of the alloy is added. In this manner, it is possible to prevent the formation of a strain-induced martensite $\varepsilon$-phase in a working step by stabilizing a $\gamma$-phase of the alloy, and to improve the plastic workability. In addition, since the Co-based alloy for a living body of the present invention does not contain Ni, there is no concern that this alloy may cause a Ni allergy in the living body.

In addition, since the Co-based alloy for a living body of the present invention has a composition obtained by adding Fe to an alloy based on Co-Cr-W, not only the plastic workability of the Co-based alloy, but also the elastic modulus and tensile strength can be improved. Moreover, since the Co-based alloy for a living body of the present invention contains W which contributes to the X-ray visibility, the X-ray visibility can be improved, so the alloy is suitable for an alloy for a stent.

The stent of the present invention uses the Co-based alloy for a living body of the present invention. Therefore, the stent does not cause a Ni allergy and has an excellent elastic modulus and tensile strength. Moreover, by containing W, the stent can exhibit excellent X-ray visibility.

BRIEF DESCRIPTION OF THE DRAWINGS

Embodiments of the present invention will now be described by way of further example only and with reference to the accompanying drawings, in which:

Figs. 1A and 1B are graphs created by plotting temperature dependency of Stacking Fault Energy (SFE) calculated using a thermodynamic model, regarding a Co-xNi alloy in Fig. 1A and a practically used Co-based alloy as well as an alloy based on Fe-Cr-Ni in Fig. 1B respectively.

Figs. 2A and 2B are graphs showing the change in Gibbs free energy calculated using a thermodynamic model, regarding a Co-xFe binary alloy (x=0 mol% to 50 mol%).

Fig. 3 is a graph created by plotting temperature dependency of stacking fault energy calculated using a thermodynamic model, regarding a Co-10W-xFe alloy and a Co-15W-xFe alloy respectively.

Fig. 4 is a view showing the calculated phases of a Co-20Cr-xW alloy.

Fig. 5A is a view showing the calculated phases of a Co-xCr-10W alloy, and Fig. 5B is a view showing the calculated phases of a Co-xCr-15W alloy.

Fig. 6 is a view showing the calculated phases of a Co-20Cr-15W-xFe alloy (x=0% by mass to 30% by mass).

Fig. 7 is a view showing the calculated phases of a Co-20Cr-10W-xFe alloy (x=0% by mass to 30% by mass).

Fig. 8 is a view collectively illustrating the theory and results of the determination of TCP-phase precipitation by using a list.

Fig. 9 is a perspective view schematically showing an example of a stent of the present invention.

Fig. 10 is a graph created by plotting the measurement results of Young's modulus, regarding the Co-10W-xFe alloy (x=0% by mass to 20% by mass) and the Co-15W-xFe alloy.

Fig. 11 is a graph showing the results of a tensile test performed on alloys of examples and comparative examples.

Fig. 12 is an X-ray diffraction pattern of the Co-10W-xFe alloy (x=0% by mass to 20% by mass).

Fig. 13 is an X-ray diffraction pattern of the Co-15W-xFe alloy (x=0% by mass to 20% by mass).

Fig. 14 is a graph showing the limiting rate of cold workability of the Co-10W-xFe alloy (x=0% by mass to 20% by mass) and the Co-15W-xFe alloy.

Fig. 15 is a graph showing a 0.2% proof strength, tensile strength (UTS), and breaking elongation of the Co-10W-xFe alloy and the Co-15W-xFe alloy.

Fig. 16 is a graph showing the comparison between a Co-10W-20Cr-xFe alloy as well as a Co-15W-20Cr-xFe alloy and other alloys, in terms of the limiting rate of cold workability.

Fig. 17 is a view showing metal structures obtained after homogenization thermal treatment is performed on Co-20Cr-10W-xFe alloys (x=0, 1, 3, 5, 10, 15, and 20% by mass) at 1250°C for 12 hours.

Fig. 18 is a view showing metal structures obtained after homogenization thermal treatment is performed on Co-20Cr-15W-xFe alloys (x=0, 1, 3, 5, 10, 15, and 20% by mass) at 1250°C for 12 hours.

Fig. 19 is a view showing the calculated phases of a Co-20Cr-15W-5Fe-xC alloy (x=0% by mass to 0.2% by mass).

Fig. 20 is a view showing the calculated phases of a Co-20Cr-15W-10Fe-xC alloy (x=0% by mass to 0.2% by mass).

Fig. 21 is a view showing the calculated phases of a Co-20Cr-15W-15Fe-xC alloy (x=0% by mass to 0.2% by mass).

Fig. 22 is a view showing the calculated phases of a Co-20Cr-10W-5Fe-xC alloy (x=0% by mass to 0.2% by mass).

Fig. 23 is a view showing the calculated phases of a Co-20Cr-10W-10Fe-xC alloy (x=0% by mass to 0.2% by mass).

Fig. 24 is a view showing the calculated phases of a Co-20Cr-10W-15Fe-xC alloy (x=0% by mass to 0.2% by mass).

DETAILED DESCRIPTION OF THE INVENTION

In order to develop a Ni-free Co-based alloy for a living body that has a high degree of strength (high tensile strength), elastic modulus, and ductility and excellent plastic workability, the present inventors conducted thorough research. As a result, the inventors found that by adding Fe, which exhibits biocompatibility and has an effect of increasing a stacking fault energy of an alloy based on Co-Cr-W, to the alloy based on Co-Cr-W, as alloy elements, the above problems can be solved.

Hereinafter, the materials science investigations that led to the present invention will be described.

First, aiming at making an ASTM F90 alloy, which is known as a material having excellent characteristics as an alloy for a living body, into a Ni-free alloy, the present inventors investigated the effect obtained by the addition of Ni to the alloy.

Ni in the Co-based alloy based on the above elements is a material added to improve plastic workability. That is, it is considered that this is because addition of Ni stabilizes the $\gamma$-phase of the fcc (face-centered cubic) structure of the Co-based alloy, and the $\varepsilon$-phase of the hcp (hexagonal close packed) structure that is a strain-induced martensite phase is not formed during the step of working, and accordingly, the alloy acquires sufficient cold workability. On the other hand, if the ASTM F90 alloy is made into a Ni-free alloy, the cold workability thereof markedly deteriorates. It is considered this is because the stability of the $\gamma$-phase deteriorates since Ni is not added, and the $\varepsilon$-phase is formed from the early stage of the working, so stress concentration is caused in the interface between the $\gamma$-phase and the $\varepsilon$-phase, whereby the alloy is broken from the interface as a starting point.

Therefore, the inventor considered that it is important to stabilize the $\gamma$-phase as the fcc structure having excellent plastic workability so as to make an alloy composition that does not form the strain-induced martensite $\varepsilon$-phase as the hcp structure during the step of working, and focus on the Stacking Fault Energy (SFE) of alloys in which phase transformation from the $\gamma$-phase to the $\varepsilon$-phase occurs, thereby further conducting examination.

Olson and Cohen have proposed the method of thermodynamically calculating the SFE of alloys in which phase transformation from the fcc (face-centered cubic) structure to the hcp (hexagonal-closed packed) structure occurs (Metall. Trans. 7A (1976), 1897-1904). According to them, if the stacking fault is regarded as thin hcp crystals, the SFE is represented as the sum of a term of volumetric energy and a term of surface energy, as shown in the following Formula (1).

$$\gamma_{SFE} = 2\rho \left( \Delta G^{\gamma \to \varepsilon} + E^{strain} \right) + 2\sigma \quad \dots (1)$$

Herein, $\Delta G^{\gamma \to \varepsilon}$, $E^{strain}$, and $\sigma$ respectively represent the change in Gibbs energy accompanied by $\gamma$-$\varepsilon$ transformation, an elastic strain energy generated when the $\varepsilon$-phase is formed in the $\gamma$-phase, and an interfacial energy in the $\gamma/\varepsilon$ boundary, and p is the atomic density per 1 mol of a $\{111\}_\gamma$ plane and can be calculated by the following Formula (2).

$$\rho = \frac{4}{\sqrt{3}} \cdot \frac{1}{a^2 N} \quad \dots (2)$$

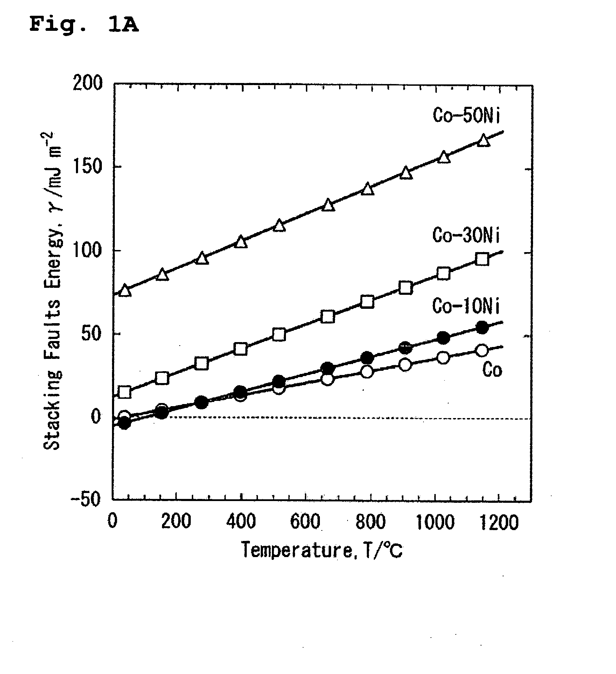

Herein, a represents a lattice constant of the fcc phase, and N represents the Avogadro constant. In the study on austenitic steel using Formula (1), volumetric change in the $\gamma \to \varepsilon$ transformation is small. Therefore, $E^{strain}$ is negligible, and likewise, a term of elastic strain energy is negligible in the case of the Co alloy. In addition, the value of $2\sigma$ practically does not show temperature dependency, and is about 15 mJ/m$^2$ in an fcc alloy. If the change in magnetic energy in $\Delta G^{\gamma \to \varepsilon}$ of cobalt is neglected, and only the change in chemical Gibbs energy is considered as the term of volumetric energy, it is possible to calculate the temperature dependency and the composition dependency of the SFE by using Thermo-Calc (manufactured by Thermo-Calc Software: ver. 4.1.3.41, database: FE ver. 6) as general purpose thermodynamics calculation software. Fig. 1A is a graph showing change of the Stacking Fault Energy (SFE) with temperature of an alloy obtained by adding Ni to Co, which is calculated using Thermo-Calc. The values of physical properties used for SFE calculation are as shown in Table 1. The temperature dependency of the interfacial energy in Formula (1) is small, and the value cannot be ascertained in transition metals. Accordingly, herein, the term of surface energy is regarded as $2\sigma^{\gamma/\varepsilon}=15$ mJm$^{-2}$ to conduct calculation.

Table 1

| Symbol | Nomenclature | value |
|---|---|---|
| $2\sigma^{\gamma/\varepsilon}$ | Surface energy of the interface $\gamma/\varepsilon$, MJ m$^{-2}$ | 15 |
| $\alpha$ | Lattice constant, nm | 0354 |
| $N$ | Avogadro's number, mol$^{-1}$ | $6.022 \times 10^{23}$ |
| $G$ | Shear modulus, GPa | 88 |
| $b_p$ | Magnitude of Burgers vectors of the partial dislocations, nm | 0.145 |
| $v$ | Poisson's ratio | 0.28 |

(continued)

| Symbol | Nomenclature | value |
|---|---|---|
| *m* | **Schmid factor** | **0.326** |
| $\gamma$ | **Stacking fault energy, mJ m$^{-2}$** | |
| | 1050° C | **29.1** |
| | 1100° C | **362** |
| | 1150° C | **433** |
| | 1200° C | **50.4** |

As shown in Fig. 1A, as the amount of Ni added to Co increases, SFE increases. Although it is known that adding Ni to Co improves ductility (plastic workability), it could be confirmed that this is because Ni is an element having an effect of increasing the stacking fault energy of the Co-based alloy.

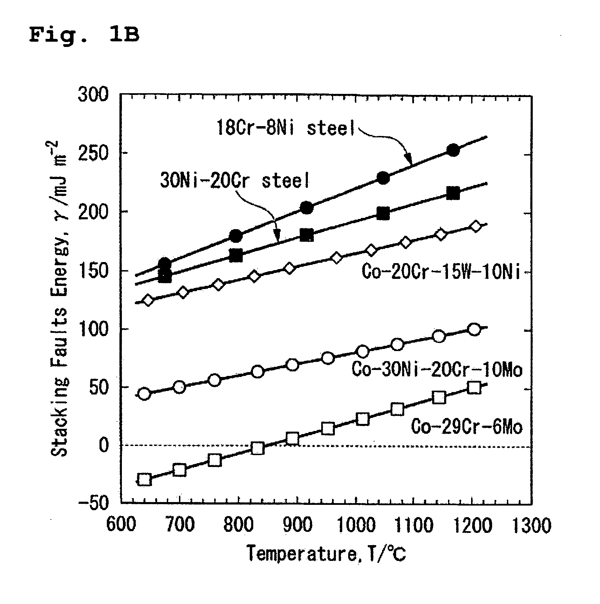

In addition, Fig. 1B shows results of calculating the change of SFE with temperature regarding various practically used Co-based alloys, by using the same calculation method as in Fig. 1A. In Fig. 1B, the Co-29Cr-6Mo alloy is an alloy that is specified by the ASTM F75 standard and used for artificial joints, the Co-30Ni-20Cr-10Mo alloy is a casting alloy of round bar materials or the like for a living body that is specified by the ASTM F562 standard, and the Co-20Cr-15W-10Ni alloy is a casting alloy that is specified by the ASTM F90 standard and applied as a tube material for a living body. Fig. 1B also describes the Thermo-Calc calculation results of SUS304 austenite-based stainless steel (18Cr-8Ni) specified by JIS and 800H high nickel steel (Fe-30Ni-20Cr).

As shown in Fig. 1B, the SFE's of the Co-based alloys are lower than that of the Fe-based alloys. Among these, the SFE of the Co-29Cr-6Mo alloy is markedly low, which is about 30 mJm$^{-2}$ to 50 mJm$^{-2}$ even at 1050°C to 1200°C. The SFE value calculated becomes negative at a temperature equal to or less than 850°C. However, in a range of temperature equal to or less than this temperature, the $\varepsilon$-phase is stabilized, and the value of $\Delta G^{\gamma \to \varepsilon}$ becomes highly negative. Therefore, it is considered that the $\gamma$-phrase of a high temperature is present metastably in such a temperature range. The Co-29Cr-6Mo as a Ni-free alloy based on Co-Cr-Mo is known to be a material having a low degree of plastic workability in which about 20% of the $\gamma$-phase as a high temperature phase remains at room temperature in addition to the $\varepsilon$-phase. It is known that if a trace of nitrogen is added to the alloy composition, almost 100% of the $\gamma$-phase remains metastably at room temperature, but due to the plastic working, phase transformation from the $\gamma$-phase to the strain-induced martensite $\varepsilon$-phase occurs, whereby cold casting workability is hindered. Accordingly, it could be confirmed that the Co-based alloy having small SFE has a low degree of plastic workability.

On the other hand, in the respective alloys including the Co-20Cr-30Ni-10Mo alloy, the ASTM F90 alloy, the SUS304 steel, and the 800H high nickel steel, the stability of the $\gamma$-phrase or the austenite phase of the fcc structure is high all the time in the temperature range shown in Fig. 1B compared to the Co-29Cr-6Mo alloy, so the SFE value of these alloys becomes large.

Among these Co-based alloys, the ASTM F90 alloy having a large SFE value has an SFE that is almost equivalent to that of practically used alloys classified as low-SFE alloys other than the Co alloy, such as austenite stainless steel. In the ASTM F90 alloy, the $\gamma$-phase is stably present until the temperature reaches room temperature, and the phase transformation to the martensite $\varepsilon$-phase induced by working hardly occurs. Consequently, the ASTM F90 alloy is known to be an alloy having excellent plastic workability at room temperature. Accordingly, it could be confirmed that the Co-based alloy having a large SFE is excellent in plastic workability.

In addition, the Co-Ni-Cr-Mo-based alloy that is represented by a Co-20Cr-30Ni-10Mo alloy having an SFE of approximately intermediate magnitude is known to exhibit a high degree of elasticity and strength. However, it is known that this alloy is inferior to the ASTM F90 alloy in terms of plastic workability, and if a large amount of Ni is added thereto, working-induced martensitic transformation is inhibited, so plastic working such as cold rolling can be performed.

From the above results, it was understood that in the Co-based alloy, the higher the SFE is, the more the plastic workability is improved, so adding an element having an effect of improving SFE to the Co-based alloy is effective for improving the plastic workability of the alloy.

In any alloys, SFE tends to increase linearly with temperature, and the value (about 30 mJm$^{-2}$) that is obtained by extrapolating the calculation result of the SUS304 steel to room temperature is close to the value reported in the related art. Moreover, Ericsson reported in the past the temperature dependency of SFE of Co- and Co-Ni-based alloys by the method using a Transmission Electron Microscope (TEM) (Acta Metall 14 (1966), 853-865), and the value is almost identical to that of the Co-Ni-based alloy obtained herein. Therefore, it is possible to judge that the SFE's and the temperature dependency in different alloys that are constructed using the same database are reliable.

In a Co-based alloy for a living body, as alloy elements to be added to a Co-Cr-W-based alloy, alloy elements showing biocompatibility and having an effect of increasing the stacking fault energy of the alloy are preferable. Among these, it is preferable to add Fe. By adding Fe, it is possible to increase the SFE of the Co-based alloy for a living body and to improve the plastic workability, the strength, the elastic modulus, and the like. The technique of specifying such alloy elements will be described below.

From the Formula (1), the size of a rough estimate of SFE of an alloy can be found by estimating $\Delta G^{\gamma \to \varepsilon}$ which represents the change in Gibbs energy accompanied by $\gamma \to \varepsilon$ transformation, that is, by estimating the difference between the free energy of the $\gamma$-phrase and the free energy of the $\varepsilon$-phase. The larger the $\Delta G^{\gamma \to \varepsilon}$, the greater the SFE. Accordingly, it is considered that when various elements are added to Co, by calculating $\Delta G^{\gamma \to \varepsilon}$, and examining the value, it is possible to specify elements effective for increasing SFE of the Co-based alloy.

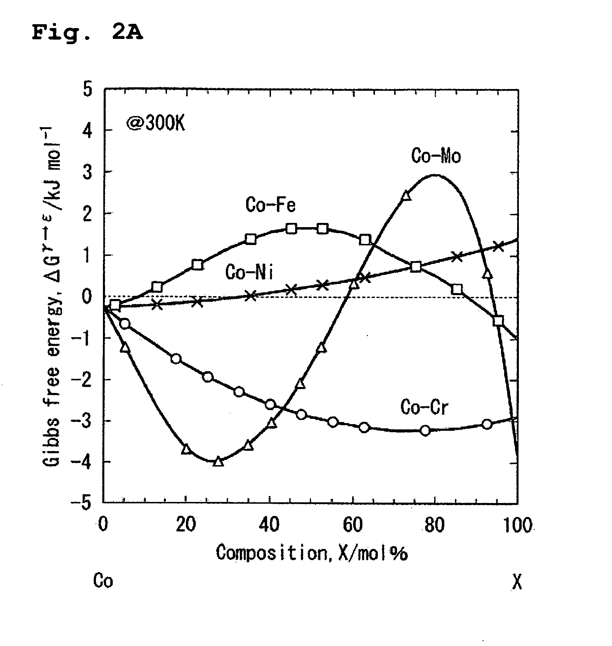

Fig. 2A shows the results obtained by calculating the composition dependency of $\Delta G^{\gamma \to \varepsilon}$ as the change in Gibbs energy accompanied by the $\gamma \to \varepsilon$ transformation that is shown when Ni, Cr, Mo, and Fe are added to Co, by using Thermo-Calc (manufactured by Thermo-Calc Software, ver. 4.1.3.41, database: FE ver. 6). As shown in Fig. 2A, when Ni is added to Co, the value of $\Delta G^{\gamma \to \varepsilon}$ increases, so it is understood that the addition of Ni increases SFE. On the other hand, when Cr is added to Co, the value of $\Delta G^{\gamma \to \varepsilon}$ decreases, so it is understood that the addition of Cr is ineffective for increasing SFE. In addition, when Mo is added to Co, the value of $\Delta G^{\gamma \to \varepsilon}$ decreases until the amount of Mo added reaches 30 mol%, but when Mo is added in an amount equal to or more than 30 mol%, the value of $\Delta G^{\gamma \to \varepsilon}$ increases. However, if practicality is taken into consideration, Mo is added in an amount of about 10 mol% to the Co-based alloy for a living body in many cases, and the value of $\Delta G^{\gamma \to \varepsilon}$ decreases when Mo is added in an amount of about 10 mol%. Therefore, it is considered that the addition of Mo decreases SFE. Moreover, when Fe is added to Co, the value of $\Delta G^{\gamma \to \varepsilon}$ increases until Fe is added in an amount of about 50 mol%, and the degree of increase is great compared to Ni. From these results, it is understood that the addition of Fe to Co can further increase SFE compared to the addition of Ni. Consequently, it is understood that the addition of Fe to Co increases the value of $\Delta G^{\gamma \to \varepsilon}$, that is, improves the plastic workability of the alloy by increasing SFE.

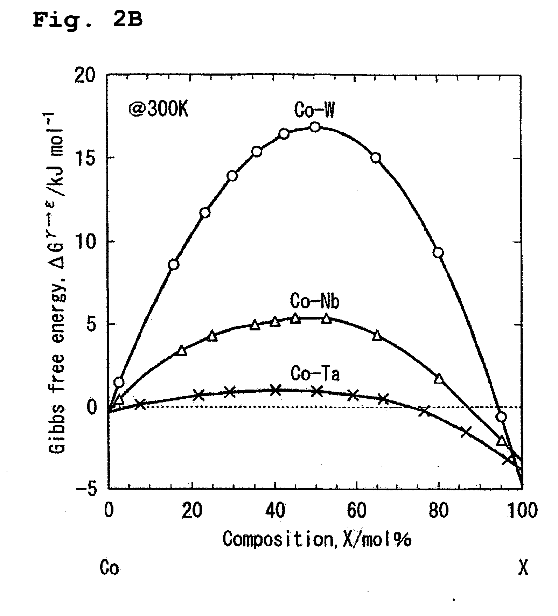

Fig. 2B shows results obtained by calculating the composition dependency of $\Delta G^{\gamma \to \varepsilon}$ as the change in Gibbs energy accompanied by the $\gamma \to \varepsilon$ transformation shown when W, Nb, and Ta are added to Co, by using Thermo-Calc. As shown in Fig. 2B, when W is added to Co, the value of $\Delta G^{\gamma \to \varepsilon}$ increases greatly until the amount of W added reaches 50 mol%, so it is understood that the addition of W to Co can increase SFE. Likewise, when Nb and Ta are added to Co, the value of $\Delta G^{\gamma \to \varepsilon}$ increases until the amount of Nb and Ta added reaches about 50 mol%, so it is understood that the addition of Nb or Ta to Co can increase SFE. When Nb or Ta is added to Co, the effect of increasing the value of $\Delta G^{\gamma \to \varepsilon}$ is small compared to when W is added to Co. However, in Ni that has the effect of increasing SFE of the Co-based alloy as shown in Fig. 1A, the degree of increase of the value of $\Delta G^{\gamma \to \varepsilon}$ is just about 1.5 kJ/mol$^{-1}$ even in a state where the Co-Ni-based alloy is almost completely substituted with Ni (adding 100 mol% of Ni). On the other hand, when Nb is added to Co at several %, the effect of increasing the value of $\Delta G^{\gamma \to \varepsilon}$ can be produced to almost the same degree as in the case of adding 100 mol% of Ni. Accordingly, it is understood that even when Ta is added in an extremely smaller amount compared to the amount of Ni added, the effect of increasing the value of $\Delta G^{\gamma \to \varepsilon}$ is produced. Consequently, the addition of Ni or Ta to Co can increase the value of $\Delta G^{\gamma \to \varepsilon}$, that is, can increase SFE, and improve the plastic workability of the alloy. Likewise, it is considered that the addition of Fe to Co can increase the value of $\Delta G^{\gamma \to \varepsilon}$ and increase the SFE of the Co-based alloy.

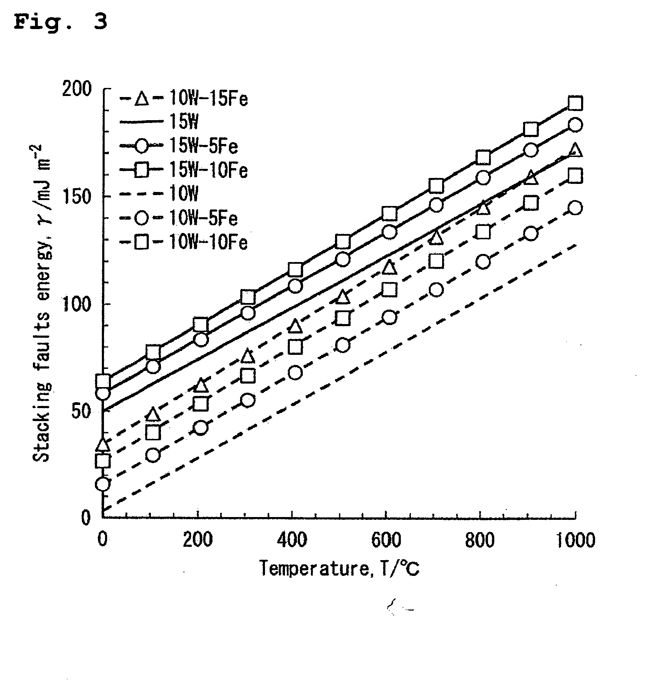

Fig. 3 shows results of calculating the phase of SFE regarding the case where Fe is added to the Co-10W alloy or the Co-15W alloy, in the same manner as in the case where SFE is calculated previously based on Fig. 1. As predicted in the description of Fig. 2, it is understood that the addition of Fe to the Co-W-based alloy increases SFE.

As the above results show, in a Co-based alloy for a living body, as alloy elements to be added to a Co-Cr-W-based alloy, alloy elements that exhibit biocompatibility and have an effect of increasing the stacking fault energy of the alloy are preferable. Among these, Fe is added since this element can make a Ni-free Co-based alloy for a living body that has a high degree of strength and elastic modulus and is excellent in the plastic workability. If Fe is added to the Co-Cr-W-based alloy, the tensile strength and Young's modulus of the Co-Cr-W-based alloy can be improved as described later in examples, and a Ni-free Co-based alloy for a living body that has a high degree of strength and elasticity and is excellent in the plastic workability can be obtained. In addition, this alloy contains W, and W is an element heavier than Co, Cr, and Ni. Therefore, overall density of the alloy increases, and even if the alloy is worked into an extremely thin alloy as an alloy for a stent, a high degree of X-ray visibility can be exhibited. Accordingly, the Co-based alloy for a living body is suitable as an alloy for a stent.

The Co-based alloy for a living body of the present invention is a Co-based alloy for a living body based on Co-Cr-W-Fe-C and having a composition of Cr: 5% by mass to 30% by mass, W: 5% by mass to 20% by mass, Fe: 1% by mass to 15% by mass, C: 0.01 % by mass to 0.15% by mass, Co as the remainder, and unavoidable impurities. The content of C more preferably ranges from 0.05% by mass to 0.10% by mass.

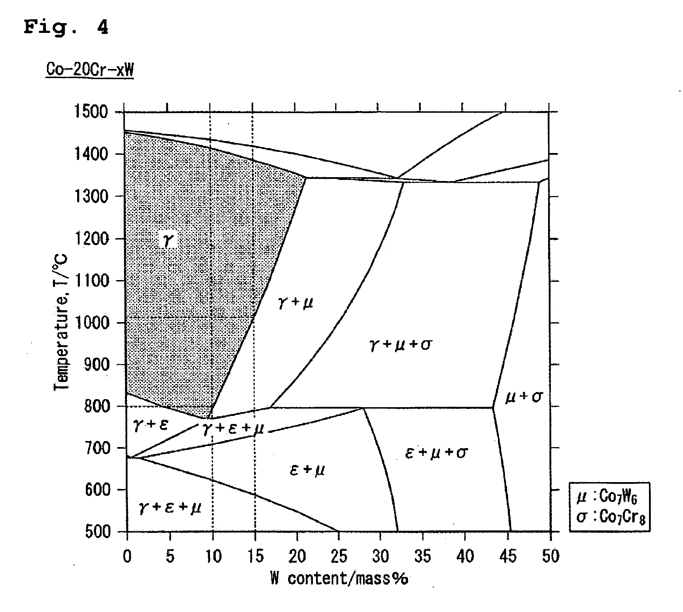

Fig. 4 is a view showing the phases of a Co-20Cr-xW alloy calculated using Thermo-Calc (manufactured by Thermo-Calc Software: ver. 4.1.3.41, database: FE ver. 6). As shown in Fig. 4, when the content of W is less than 20% by mass, the $\gamma$-phase of the fcc structure is stabilized. As described above, considering the fact that W produces an effect of improving the plastic workability by increasing SFE of the Co-based alloy, the content of W is 5% by mass to 20% by mass and more preferably 5% by mass to 15% by mass. If the amount of W added exceeds 20% by mass, there is a possibility that the μ-phase ($CO_7W_6$), the σ-phase ($Co_7Cr_8$), or the like may be formed, and the mechanical characteristics may deteriorate. In addition, the addition of W makes it possible to improve the density and solution strengthening in the alloy and improve the X-ray visibility.

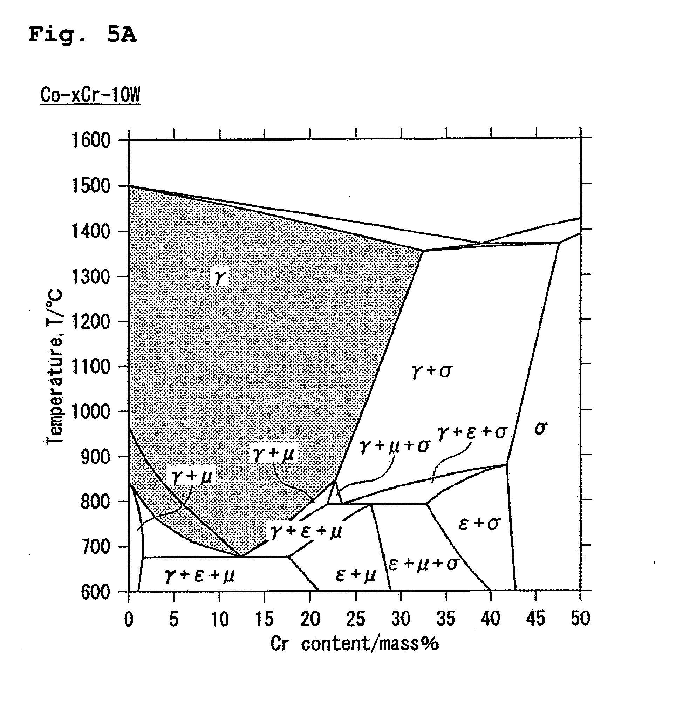

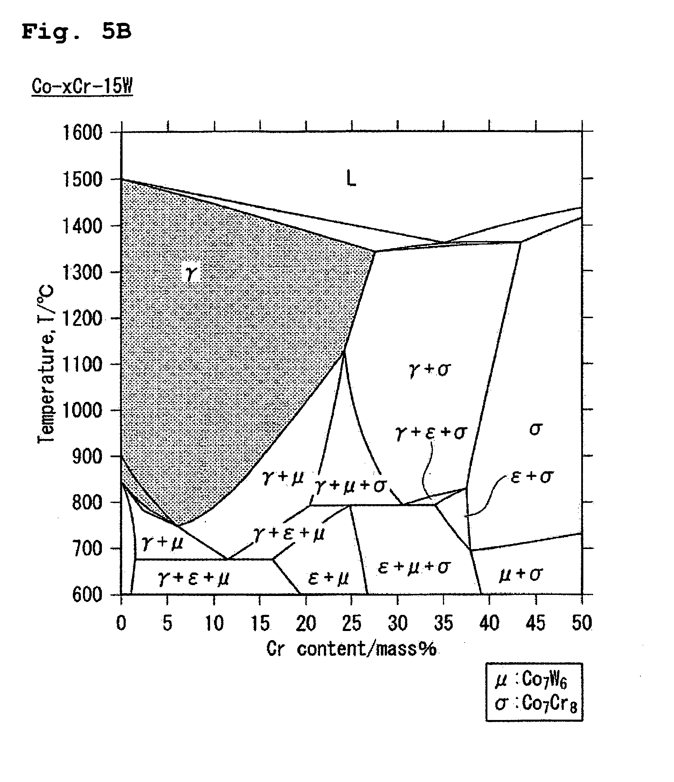

Fig. 5A is a view showing the phases of a Co-xCr-10W alloy calculated using Thermo-Calc, and Fig. 5B is a view showing the phases of a Co-xCr-15W alloy calculated using Thermo-Calc. As shown in Figs. 5A and 5B, the content of Cr is 5% by mass to 30% by mass, since the γ-phase of the fcc structure is stabilized and the phase transformation caused in the step of working is inhibited with this content. In view of improving corrosion resistance of the alloy, the content of Cr is more preferably 16% by mass to 25% by mass. If the amount of Cr added exceeds 30% by mass, there is a possibility that the μ-phase ($Co_7W_6$), the σ-phase ($Co_7Cr_8$), or the like may be formed, and the mechanical characteristics may deteriorate.

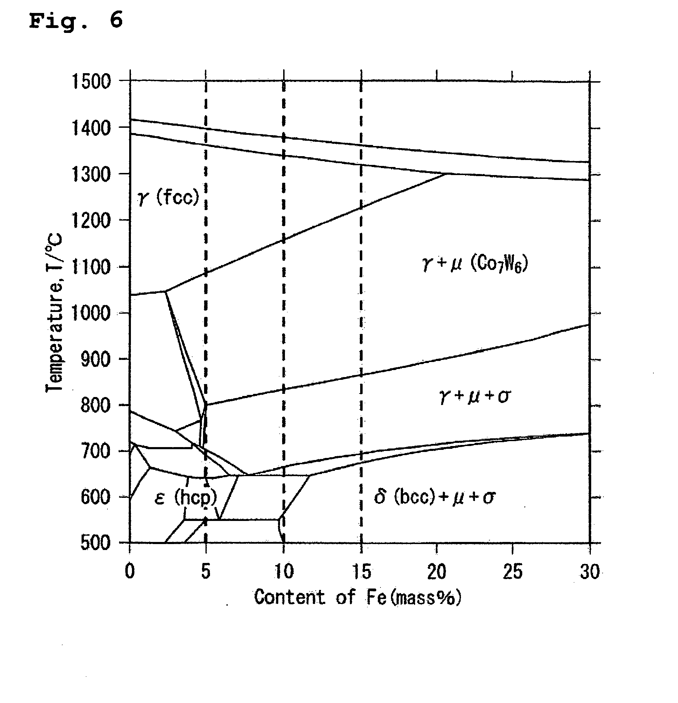

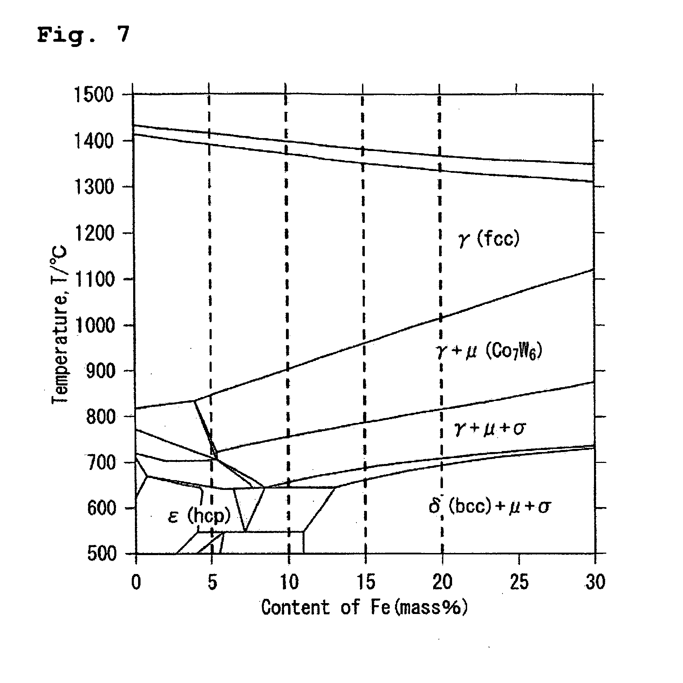

Fig. 6 is a view showing the phases of a Co-20Cr-15W-xFe alloy calculated using Thermo-Calc, and Fig. 7 is a view showing the phases of a Co-20Cr-10W-xFe alloy calculated using Thermo-Calc. As shown in Figs. 6 and 7, in the case of the Co-20Cr-15W-xFe alloy, the content of Fe can be set to 20% by mass or less and is desirably set to 15% by mass or less, since the γ-phase of the fcc structure is stabilized and the phase transformation in the step of process is inhibited with this content. Moreover, in the case of the Co-20Cr-10W-xFe alloy, the content of Fe can be set within a range of 30% by mass or less and is preferably set to 20% by mass or less.

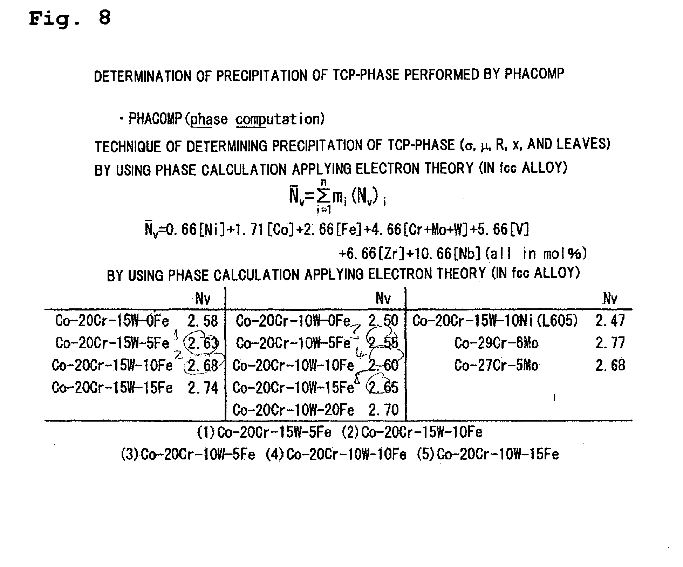

Next, regarding the alloy based on the above elements, the determination results of the precipitation of the TCP-phase (topologically close packed phase) obtained by PHACOMP (Phase Computation) will be described based on the calculation formula and the calculation results shown in Fig. 8.

PHACOMP refers to a technique of determining precipitation (in an FCC alloy) of the TCP-phase (σ, μ, R, x, Leaves) by using phase calculation applying electron theory, and can be represented by the following Formulae (3) and (4). It is said that in the Co-based alloy, TCP is easily precipitated with Nv≥2.7. Nv represents the Average electron-hole number.

As shown in the determination results of the TCP-phase precipitation shown in Fig. 8, Nv of the Co-20Cr-15W-15Fe alloy is 2.74, and Nv of the Co-20Cr-10W-20Fe alloy is 2.7. Accordingly, precipitates are considered to be easily formed. However, in any of alloys having other compositional ratios, the value of Nv are less than 2.7.

In the examples described later, the alloy composition of examples and the alloy composition of comparative examples were selected based on the above results.

$$\overline{N}_v = \sum_{i=1}^{n} m_i (N_v)_i \quad \dots (3)$$

$$\overline{N}_v = 0.66[Ni] + 1.71[Co] + 2.66[Fe] + 4.66[Cr + Mo + W] + 5.66[V] + 6.66[Zr] + 10.66[Nb] \quad \dots (4)$$

(all values are based on mol%)

Fe as an alloy element that exhibits biocompatibility and has an effect of increasing the Stacking Fault Energy (SFE) of a Co-Cr-W-based alloy is added to the Co-Cr-W-based alloy. In this manner, the γ-phrase of the alloy is stabilized, and the formation of strain-induced martensite ε-phase in the step of working is prevented, whereby the plastic workability can be improved. In addition, since the Co-based alloy for a living body does not contain Ni, there is no concern that the alloy may cause a Ni allergy in the living body.

Moreover, since the Co-based alloy for a living body has a composition obtained by adding Fe to the Co-Cr-W-based alloy, not only the plastic workability of the Co-based alloy, but also the elastic modulus and tensile strength can be improved. Furthermore, since the Co-based alloy for a living body contains W, the X-ray visibility of the alloy can be improved, so the alloy is suitable as an alloy for a stent.

The Co-based alloy for a living body of the present invention is an alloy having a composition of Co-Cr-W-Fe-C.

In the case of the alloy based on the above elements, the composition of Cr, W, and Fe is in the same range as in the alloy based on Co-Cr-W-Fe described above, and C is further contained in the alloy in a range of from 0.01 % by mass to 0.15% by mass. The content of C is more preferably 0.05% by mass to 0.10% by mass.

In the alloy based on Co-Cr-W-Fe, when the TCP-phase which is hard and brittle, such as the μ-phase or the σ-phase, is precipitated, there is a concern that the alloy may be cracked during hot casting due to the presence of these brittle precipitates. If the composition of Cr, W, and Fe is specified as being within the above range, and the C is contained in the alloy within the above range, the precipitation of the TCP-phase can be inhibited, whereby the cracking caused during hot casting can be inhibited. Consequently, the plastic workability can be improved.

Next, the stent of the present invention will be described.

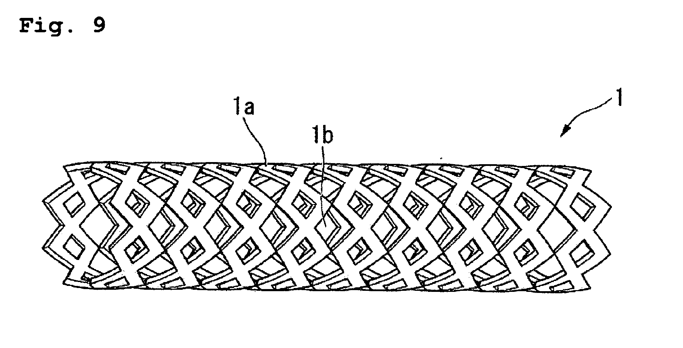

The stent of the present invention is used by being inserted into stenosed portions in blood vessels, bile ducts, and the like in the living body so as to dilate the lumens and maintain the luminal diameter, and uses the Co-based alloy for a living body of the present invention. Fig. 9 is a perspective view schematically showing an example of the stent according to the present invention. A stent 1 shown in Fig. 9 has a cylindrical structure constituted such that the stent is transformable while expanding and contracting the diameter by a frame 1a. The stent 1 has a mesh-like structure that has a plurality of approximately rhombic notch portions 1 b in the side forming the cylindrical structure. If a stress is applied to the stent, the stent can be transformed by expanding or contracting in diameter. The stent 1 shown in Fig. 9 is a balloon-expanding stent. While a balloon catheter is fixed inside the cylindrical stent 1, the stent 1 is inserted into a target site, and then the balloon is expanded to plastically transform the stent, whereby the stent can be pressed against and fixed to the inner surface of the target site.

As a method of preparing the stent 1 having such a structure, for example, a pipe with a length, a diameter, a wall thickness, and the like having desired dimensions is formed using the Co-based alloy for a living body of the present invention, and then the side of the pipe is partially cut out by a cutting process or the like to form a plurality of notch portions 1 b, whereby the stent 1 can be prepared.

In Fig. 9, as the shape of the frame 1a of the stent 1 that can be transformed by expanding or contracting the diameter, a mesh-like frame is shown for example. However, the present invention is not limited to this example, and for example, the stent can shaped into those known in the related art, such as a coil shape and a multiple spiral shape.

Using the Co-based alloy for a living body of the present invention described above, the stent of the present invention does not cause a Ni allergy and has an excellent elastic modulus and tensile strength. In addition, for the Fe-added Co-based alloy for a living body of the present invention, Fe is selected as an alternative element for Ni, whereby an effect of improving workability (ductility) is obtained.

Examples

Examples 1 to 12, Comparative Examples 1 and 2 and the "example in related art" described below do not form part of the claimed invention.

Alloys of Examples 1 to 12 and Comparative Examples 1 and 2, and example alloys in the related art that contain the respective elements as the component composition shown in the following Table 2 were prepared in the following manner.

By using a high-frequency vacuum induction melting furnace, the respective elements were mixed in the component composition shown in Table 2 and dissolved, thereby obtaining a molten alloy. The molten alloy was cast into a metallic mold under an Ar atmosphere of 800 Pa, and the furnace was cooled. The ingot was shaped into a cylinder having sizes of a diameter of the upper portion of 80 mm, a diameter of the lower portion of 70 mm, and a height of 120 mm and a mass of 6 kg. Thereafter, in order to prevent solidification segregation, homogenization thermal treatment was performed on the ingot at 1220°C for 10 hours under an Ar atmosphere by using a high-temperature high-vacuum furnace manufactured by TOKYO VACUUM, Co, Ltd., and then the furnace was cooled to room temperature, thereby preparing the respective alloys. During the homogenization thermal treatment, the rate of temperature increase was set to 10°C/min, and the cooling rate was set to 10°C/min.

Table 2

| Sample | | Component composition (% by mass) | | | | |
|---|---|---|---|---|---|---|
| | | Co | Cr | W | Fe | Ni |
| Comparative Example 1 | Co-20Cr-10W | Remainder | 20 | 10 | 0 | |
| Example 1 | Co-20Cr-10W-1Fe | Remainder | 20 | 10 | 1 | |
| Example 2 | Co-20Cr-10W-3Fe | Remainder | 20 | 10 | 3 | |
| Example 3 | Co-20Cr-10W-5Fe | Remainder | 20 | 10 | 5 | |
| Example 4 | Co-20Cr-10W-10Fe | Remainder | 20 | 10 | 10 | |
| Example 5 | Co-20Cr-10W-15Fe | Remainder | 20 | 10 | 15 | |
| Example 6 | Co-20Cr-10W-20Fe | Remainder | 20 | 10 | 20 | |
| Comparative Example 2 | Co-20Cr-15W | Remainder | 20 | 15 | 0 | |
| Example 7 | Co-20Cr-15W-1Fe | Remainder | 20 | 15 | 1 | |

(continued)

| Sample | | Component composition (% by mass) | | | | |
|---|---|---|---|---|---|---|
| | | Co | Cr | W | Fe | Ni |
| Example 8 | Co-20Cr-15W-3Fe | Remainder | 20 | 15 | 3 | |
| Example 9 | Co-20Cr-15W-5Fe | Remainder | 20 | 15 | 5 | |
| Example 10 | Co-20Cr-15W-10Fe | Remainder | 20 | 15 | 10 | |
| Example 11 | Co-20Cr-15W-15Fe | Remainder | 20 | 15 | 15 | |
| Example 12 | Co-20Cr-15W-20Fe | Remainder | 20 | 15 | 20 | |
| Example in related art | Co-20Cr-15W-10Ni | Remainder | 20 | 15 | 0 | 10 |

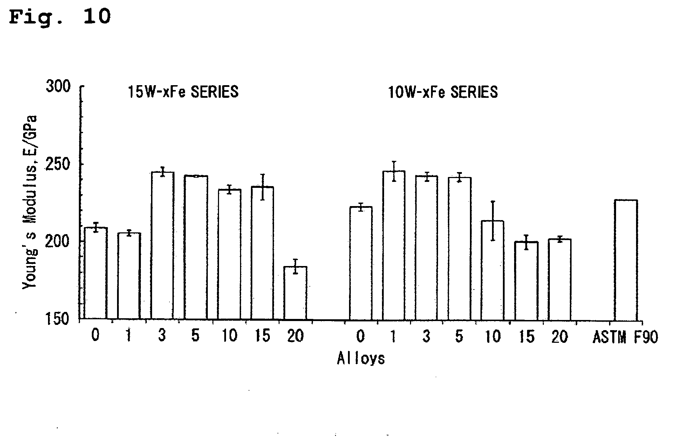

Fig. 10 shows the measurement results of the elastic modulus (Young's modulus) of the alloys of examples and comparative examples and the example alloys in the related art. From the results, it is understood that the addition of Fe to the Co-Cr-W-based alloy improves the elastic modulus. When Fe was added to the Co-20Cr-15W alloy, the elastic modulus was improved particularly in a range of 3% by mass to 15% by mass. In addition, when Fe was added to the Co-20Cr-10W alloy, the elastic modulus was improved particularly in a range of 1% by mass to 5% by mass. In these ranges, it was possible to obtain an excellent elastic modulus superior to that of the ASTM F90 alloy used as an example alloy in the related art for comparison. Moreover, although there is a concern that the ASTM F90 alloy may cause a Ni allergy since this alloy contains Ni, none of the alloys of examples and comparative examples contains Ni, so these alloys do not cause the problem of the Ni allergy.

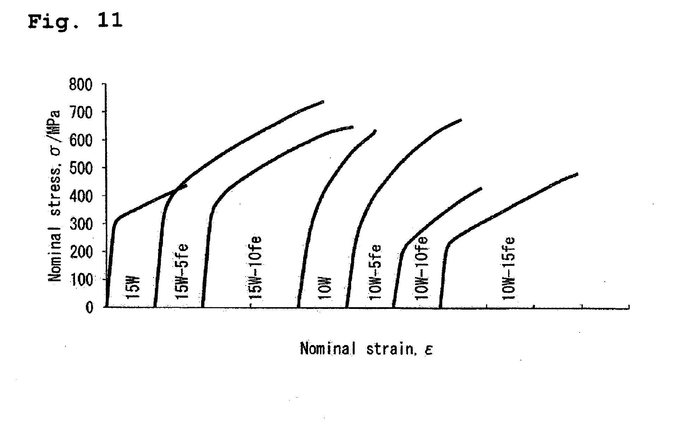

Fig. 11 shows the results obtained by performing isothermal forging on the respective alloys of examples and comparative examples and the example alloys in the related art at room temperature, performing uniform heating treatment for 12 hours, and then conducting a tensile test on the respective alloys.

From the results shown in Fig. 11, it is understood that the addition of Fe to the Co-20Cr-15W alloy greatly improves the tensile strength and the ductility. On the other hand, it is understood that even if Fe is added to the Co-20Cr-10W alloy, neither of the tensile strength and ductility is improved compared to when Fe is not yet added. It is considered that this is because since a non-isothermal martensite structure is frequently observed in the alloy based on such elements as shown in pictures of metal structures described later, this structure influences the ductility.

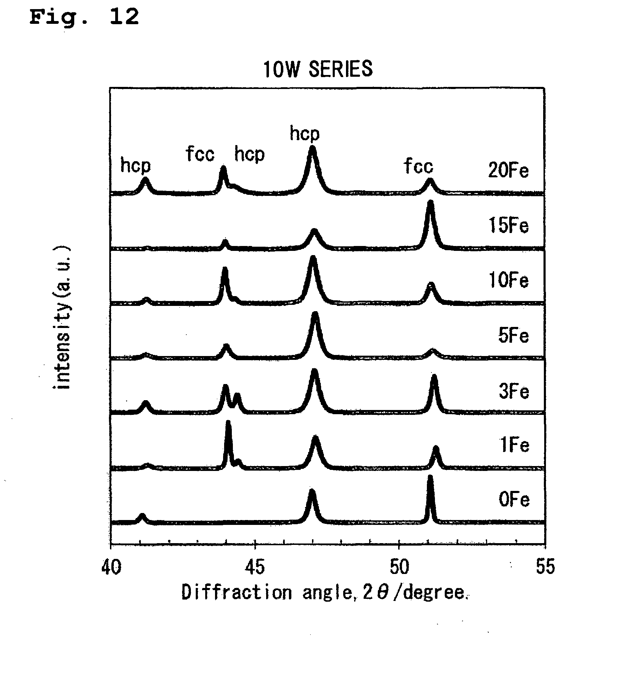

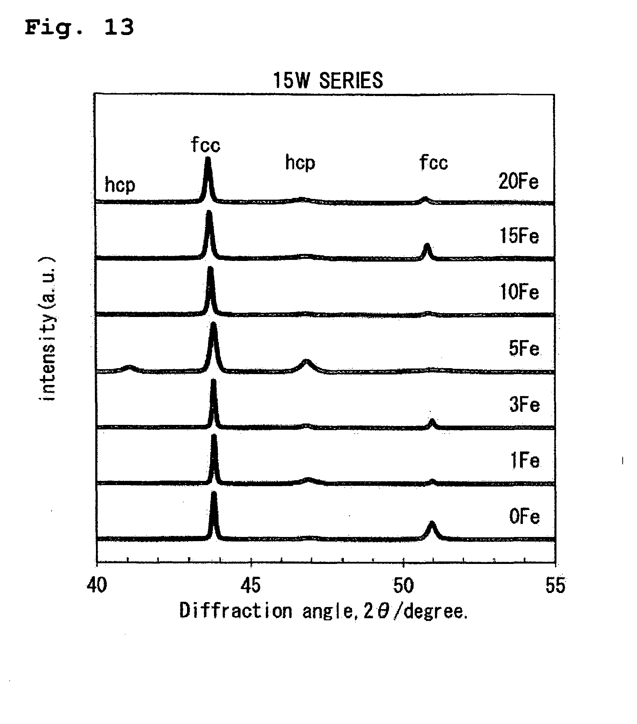

Fig. 12 shows X-ray diffraction patterns obtained after homogenization thermal treatment was performed on the alloy samples having the respective compositions in the case where Fe was added to the Co-20Cr-10W alloy, and Fig. 13 shows X-ray diffraction patterns obtained after homogenization thermal treatment was performed on the alloy samples having the respective compositions in the case where Fe was added to the Co-20Cr-15W alloy. Comparing the patterns with each other, these alloys have a double-phase structure in which a peak of the ε-phase of the hcp structure coexists with a peak of the γ-phase of the fcc structure. It is understood that the addition of Fe to the Co-20Cr-15W alloy makes this alloy have a double-phase structure in which a peak of the γ-phase coexists with a peak of the γ-phase of the fcc structure. It is also understood that the addition of Fe to the Co-20Cr-15W alloy increased the ratio of the γ-phase from 69% to 97% (converted from the diffraction peak), so the γ-phase could be stabilized. On the other hand, even when Fe was added to the Co-20Cr-10W alloy, stabilization of the γ-phase was not marked. This means that increasing the amount of W added contributes more to the stabilization of the γ-phase, compared to the effect of stabilizing the γ-phase that is produced by adding Fe to the Co-Cr-W-based alloy.

Accordingly, in the Co-Cr-W-Fe-based alloy, it is preferable to calculate the γ-phase stabilization effect produced by the addition of Fe and the γ-phase stabilization effect produced by the addition of W, and to set the amount of each of Fe and W within a suitable range.

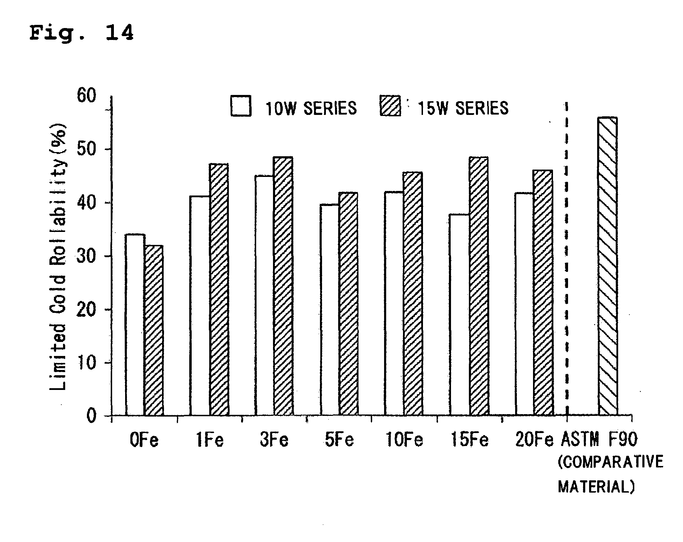

Fig. 14 shows the limiting rate of cold workability of the alloy samples having the respective composition in the case where Fe was added to the Co-20Cr-10W alloy and the Co-20Cr-15W alloy. The greater value shown in Fig. 14 indicates that cold working could be more reliably performed without causing a defect. In the limiting rate of cold workability, the Fe-added samples showed a superior rate of workability compared to the samples to which Fe was not added, regardless of the amount of the Fe added. In addition, it is understood that the Fe-added samples had ductility equivalent to that of the L605 alloy.

Regarding the cold workability, from the results shown in Fig. 14, it is understood that the workability can be improved by adding Fe in a range of 1% by mass to 20% by mass to the Co-Cr-W-based alloy.

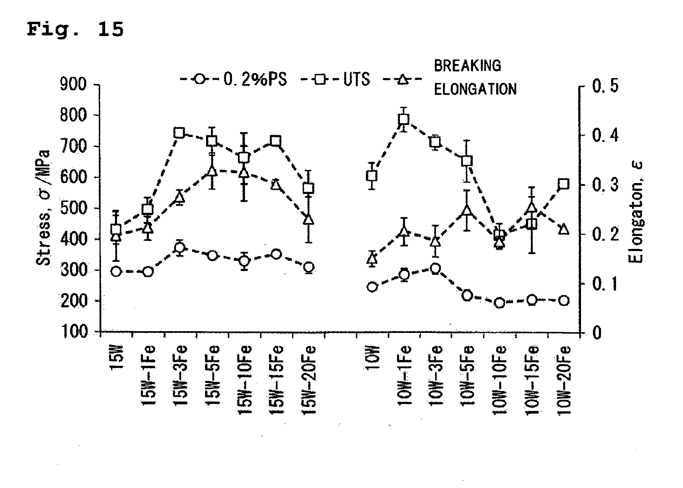

Fig. 15 collectively shows the measurement results of 0.2% proof strength, tensile strength (UTS), and breaking elongation of the alloy samples having the respective composition in the case where Fe was added to the Co-20Cr-10W alloy and the Co-20Cr-15W alloy. Comparing the results with each other, it is understood that in the Co-20Cr-10W alloy, the effect of improving the tensile strength becomes strong when Fe is added in a range of 1% by mass to 5% by mass, and in the Co-20Cr-15W alloy, the effect of improving the tensile strength becomes strong when Fe is added in a range of 3% by mass to 15% by mass.

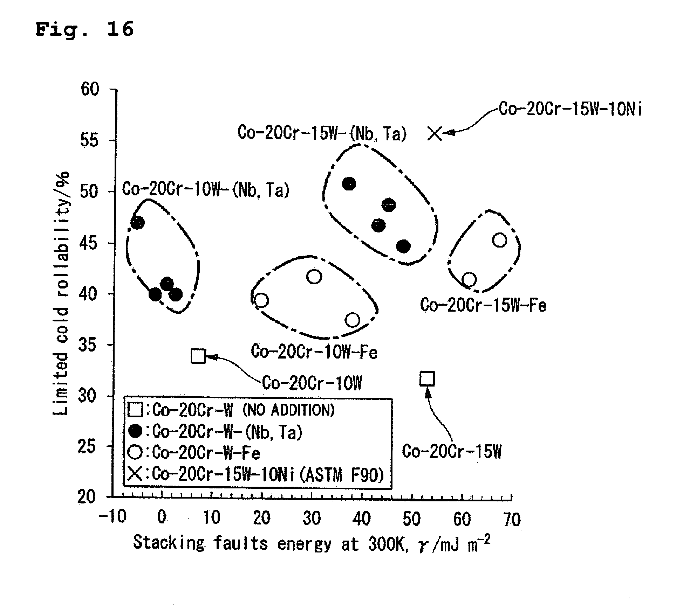

Fig. 16 shows the relationship between SFE and the alloy samples obtained by adding Fe to the Co-20Cr-10W alloy and the Co-20Cr-15W alloy as well as the alloys obtained by adding Nb or Ta to the above alloys. It is understood that though the alloys obtained by adding Nb or Ta to the Co-20Cr-10W alloy and the Co-20Cr-15W alloy are excellent alloys, if Fe is added thereto, the alloys can obtain excellent cold workability while exhibiting an SFE in a range slightly different from the value of the SFE obtained from alloys to which Nb or Ta is added.

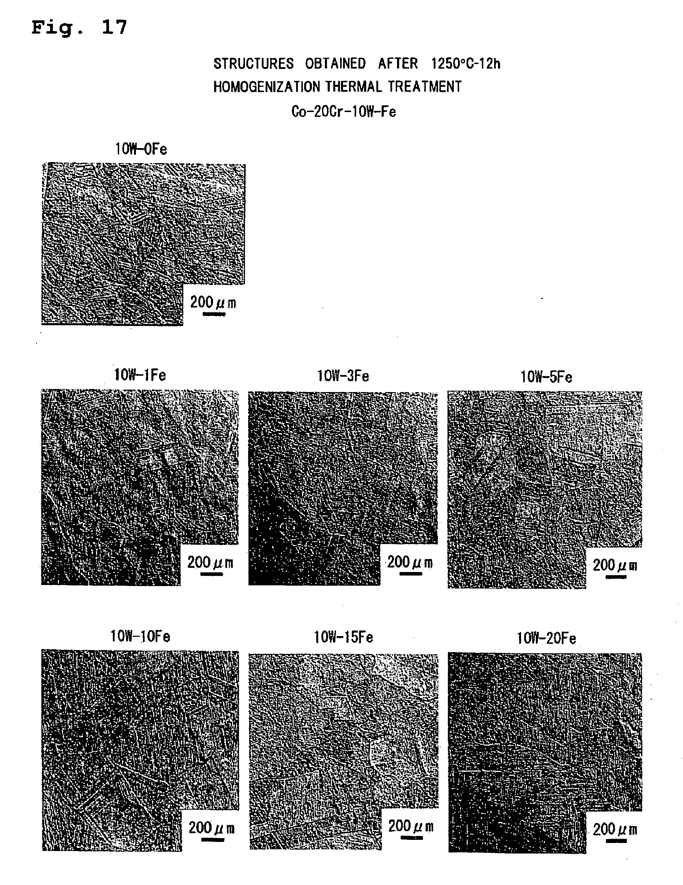

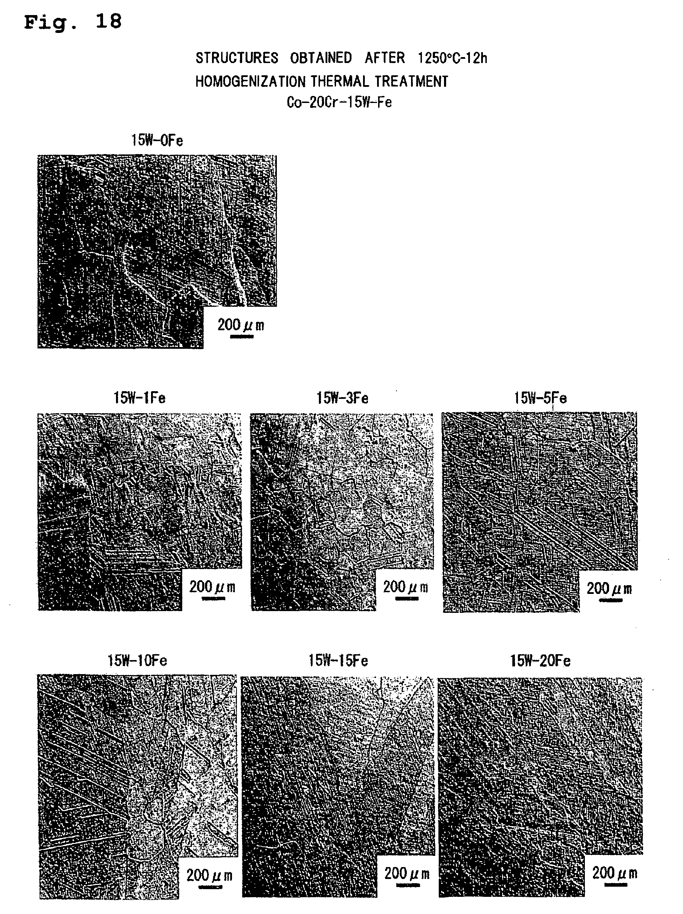

Figs. 17 and 18 show optical microscopic structures of various alloys having undergone homogenization thermal treatment for 12 hours after being subjected to casting at a constant temperature of 1250°C. Fig. 17 shows metal structures of the Co-20Cr-10W alloy, the Co-20Cr-10W-1Fe alloy, the Co-20Cr-10W-3Fe alloy, the Co-20Cr-10W-5Fe alloy, the Co-20Cr-10W-10Fe alloy, the Co-20Cr-10W-15Fe alloy, and the Co-20Cr-10W-20Fe alloy respectively. Fig. 18 shows metal structures of the Co-20Cr-15W alloy, the Co-20Cr-15W-1Fe alloy, the Co-20Cr-15W-3Fe alloy, the Co-20Cr-15W-5Fe alloy, the Co-20Cr-15W-10Fe alloy, the Co-20Cr-15W-15Fe alloy, and the Co-20Cr-15W-20Fe alloy respectively.

Comparing Fig. 17 with Fig. 18, the non-isothermal martensite structure is frequently observed in the metal structure of the Co-20Cr-10W-xFe alloy shown in Fig. 17. From this result, it is considered that since the effect obtained by the addition of W is weaker in the Co-20Cr-10W-xFe alloy than in the Co-20Cr-15W-xFe alloy, the $\gamma$-phase stabilization effect is diminished, and the ductility improving effect is weak as described above based on Fig. 11. However, it is considered that in order to control the structure of the metal structure of the alloys based on such elements, if hot working and warm working are performed to miniaturize the crystal grains, the problem of ductility improvement can be solved.

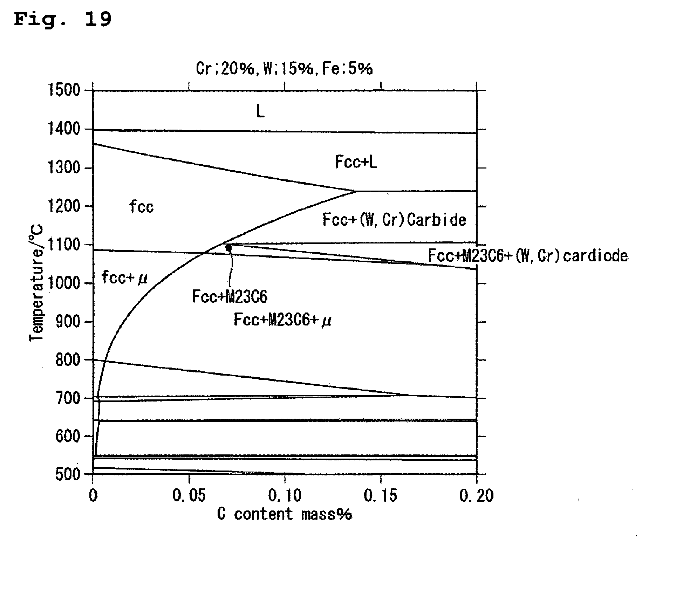

Fig. 19 is a view showing the phases of a Co-20Cr-15W-5Fe-xC alloy according to the present invention calculated using Thermo-Calc (manufactured by Thermo-Calc Software: ver. 4.1.3.41, database: FE ver. 6). As shown in Fig. 19, it is understood that when C is added in such an amount that the content of C falls into a range of 0.01 % by mass to 0.15% by mass, the TCP-phase represented as the $\mu$-phase can be reduced by being partially substituted with a carbide of M23C6 such that the $\gamma$-phase of the fcc (face-centered cubic) structure can be stabilized, and accordingly, the improvement of plastic workability such as reduction of cracking in hot casting can be realized. In addition, it is understood that the content of C more preferably ranges from 0.05% by mass to 0.10% by mass within the above range.

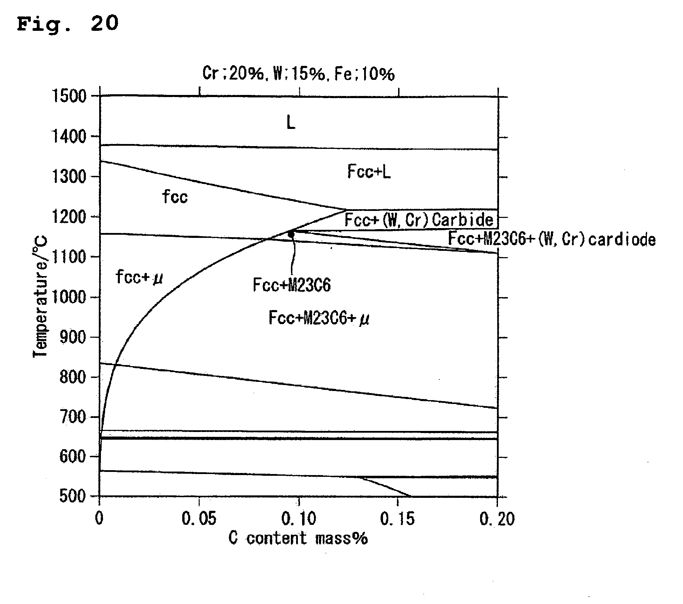

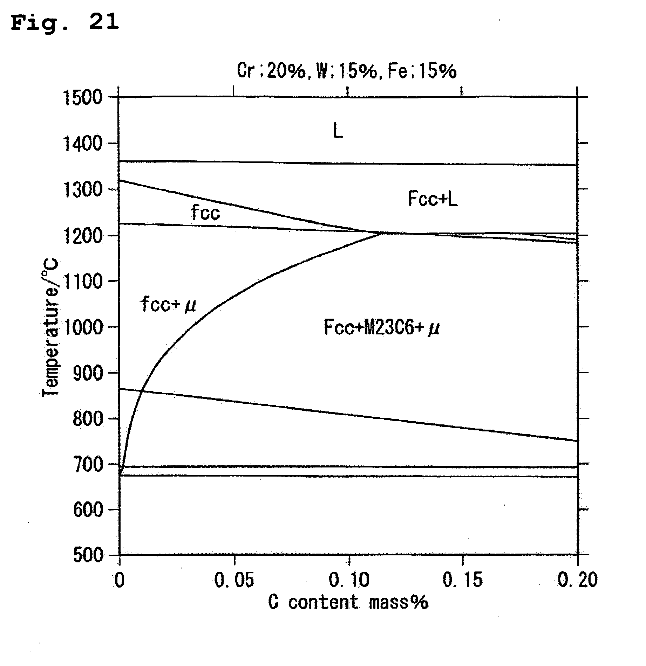

Fig. 20 is a view showing the phases of a Co-20Cr-15W-10Fe-xC alloy according to the present invention calculated in the same manner as above, and Fig. 21 is a view showing the phases of a Co-20Cr-15W-15Fe-xC alloy according to the present invention calculated in the same manner as above. Similarly to Fig. 19, the TCP-phase as the $\mu$-phase can be reduced such that the $\gamma$-phase can be stabilized in the results shown in Figs. 20 and 21. Accordingly, the content of C preferably ranges from 0.01 % by mass to 0.15% by mass and more preferably ranges from 0.05% by mass to 0.10% by mass.

From these views showing the phases of alloys, it is understood that if the content of C is set to be in the above range, the TCP-phase can be reduced, and accordingly, the improvement of plastic workability such as the reduction of cracking in hot casting can be realized.

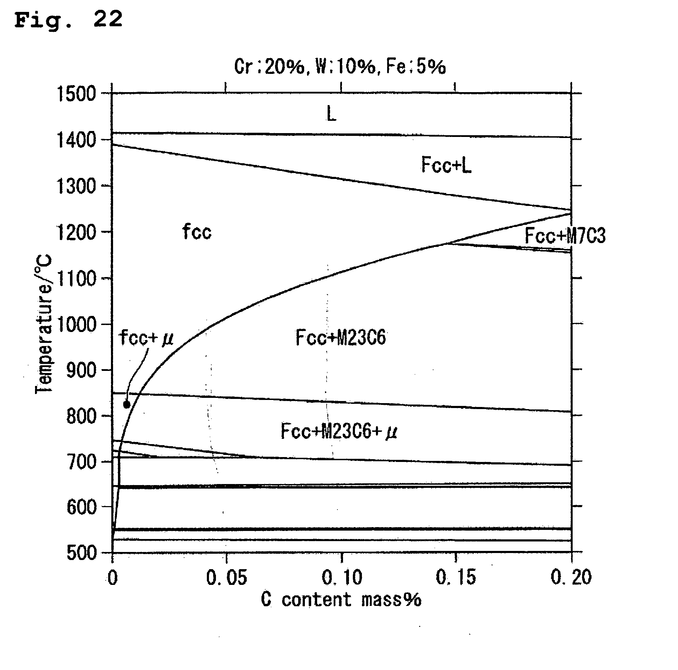

Fig. 22 is a view showing the phases of a Co-20Cr-10W-5Fe-xC alloy according to the present invention calculating using Thermo-Calc (manufactured by Thermo-Calc Software: ver. 4.1.3.41, database: FE ver. 6). As shown in Fig. 22, it is understood that when C is added in such an amount that the content of C falls into a range of 0.01 % by mass to 0.15% by mass, the TCP-phase represented as the $\mu$-phase can be reduced by being partially substituted with a carbide of M23C6 such that the $\gamma$-phase of the fcc (face-centered cubic) structure can be stabilized, and accordingly, the improvement of plastic workability such as reduction of cracking in hot casting can be realized. In addition, it is understood that the content of C preferably ranges from 0.05% by mass to 0.10% by mass within the above range.

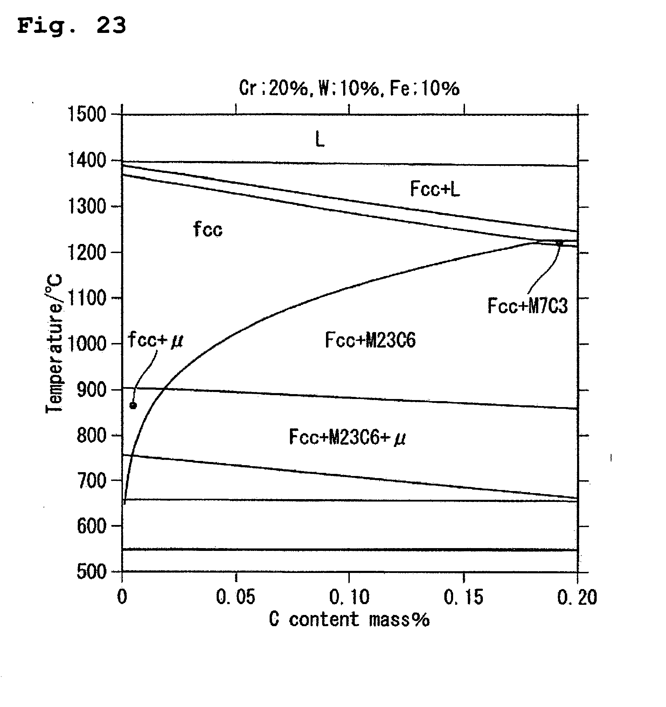

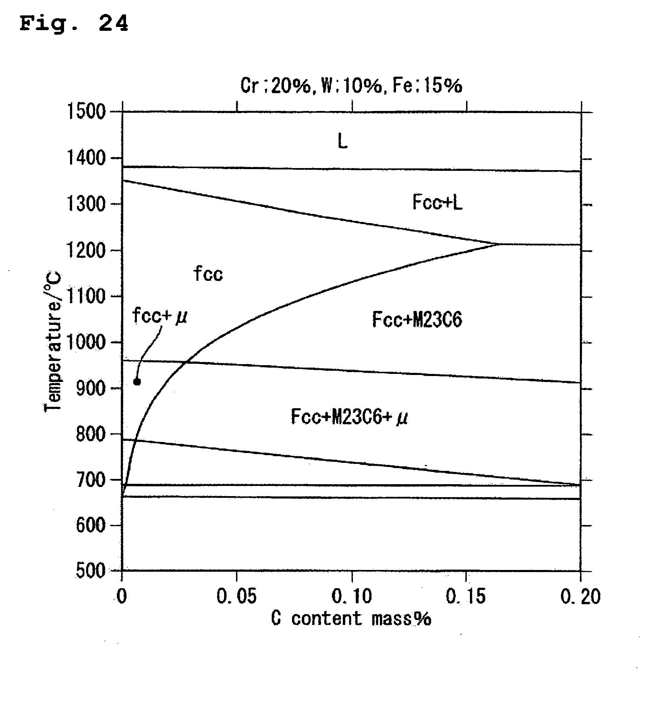

Fig. 23 is a view showing the phases of a Co-20Cr-10W-10Fe-xC alloy according to the present invention calculated in the same manner as above, and Fig. 24 is a view showing the phases of a Co-20Cr-10W-15Fe-xC alloy according to the present invention calculated in the same manner as above. Similarly to Fig. 22, in the results shown in Figs. 23 and 24, the TCP-phase represented by the symbol $\mu$ can be reduced such that the $\gamma$-phase can be stabilized. Accordingly, the content of C preferably ranges from 0.01 % by mass to 0.15% by mass and more preferably ranges from 0.05% by mass to 0.10% by mass.

From these views showing the phases of alloys, it is understood that if the content of C is set to be in the above range, the TCP-phase can be reduced, and accordingly, the improvement of plastic workability such as the reduction of cracking in hot casting can be realized.

The foregoing description has been given by way of example only and it will be appreciated by a person skilled in the art that modifications can be made without departing from the scope of the present invention.

**Claims**

1. A Co-based alloy for a living body based on Co-Cr-W-Fe-C, comprising a composition of Cr: 5% by mass to 30% by mass, W: 5% by mass to 20% by mass, Fe: 1% by mass to 15% by mass, C: 0.01% by mass to 0.15% by mass, Co as the remainder, and unavoidable impurities.

2. The Co-based alloy for a living body based on Co-Cr-W-Fe-C according to Claim 1, wherein the content of W is 5% by mass to 10% by mass, and the content of Fe is 1 % by mass to 5% by mass.

3. The Co-based alloy for a living body based on Co-Cr-W-Fe-C according to Claim 1, wherein the content of W is 11 % by mass to 20% by mass, and the content of Fe is 3% by mass to 15% by mass.

4. A stent comprising the Co-based alloy for a living body according to any one of Claims 1 to 3.


**Patentansprüche**

1. Co-basierte Legierung für einen lebenden Körper auf der Basis von Co-Cr-W-Fe-C, die eine Zusammensetzung aus Cr: 5 Masse-% bis 30 Masse-%, W: 5 Masse-% bis 20 Masse-%, Fe: 1 Masse-% bis 15 Masse-%, C: 0,01 Masse-% bis 0,15 Masse-%, Co als der Rest, und unvermeidliche Verunreinigungen umfasst.

2. Co-basierte Legierung für einen lebenden Körper auf der Basis von Co-Cr-W-Fe-C nach Anspruch 1, wobei der Gehalt an W 5 Masse-% bis 10 Masse-% beträgt und der Gehalt an Fe 1 Masse-% bis 5 Masse-% beträgt.

3. Co-basierte Legierung für einen lebenden Körper auf der Basis von Co-Cr-W-Fe-C nach Anspruch 1, wobei der Gehalt an W 11 Masse-% bis 20 Masse-% beträgt und der Gehalt an Fe 3 Masse-% bis 15 Masse-% beträgt

4. Stent, der eine Co-basierte Legierung für einen lebenden Körper nach einem der Ansprüche 1 bis 3 umfasst.


**Revendications**

1. Alliage à base de Co pour un corps vivant sur la base de Co-Cr-W-Fe-C, comprenant une composition de 5% en masse à 30% en masse de Cr, de 5% en masse à 20% en masse de W, de 1% en masse à 15% en masse de Fe, de 0,01% en masse à 0,15% en masse de C, le reste étant du Co, ainsi que des impuretés inévitables.

2. Alliage à base de Co pour un corps vivant sur la base de Co-Cr-W-Fe-C selon la revendication 1, dans lequel la teneur en W est de 5% en masse à 10% en masse, et la teneur en Fe étant de 1% en masse à 5% en masse.

3. Alliage à base de Co pour un corps vivant sur la base de Co-Cr-W-Fe-C selon la revendication 1, dans lequel la teneur en W est de 11% en masse à 20% en masse, et la teneur en Fe étant de 3% en masse à 15% en masse.

4. Endoprothèse comprenant l'alliage à base de Co pour un corps vivant selon l'une quelconque des revendications 1 à 3.

Fig. 1A

Fig. 1B

Fig. 2A

Fig. 2B

Fig. 3

Fig. 4

Co-20Cr-xW

## Fig. 5A

Co-xCr-10W

## Fig. 5B

Co-xCr-15W

Fig. 6

## Fig. 7

# Fig. 8

DETERMINATION OF PRECIPITATION OF TCP-PHASE PERFORMED BY PHACOMP

· PHACOMP (phase computation)

TECHNIQUE OF DETERMINING PRECIPITATION OF TCP-PHASE (σ, μ, R, x, AND LEAVES)
BY USING PHASE CALCULATION APPLYING ELECTRON THEORY (IN fcc ALLOY)

$$\overline{N}_v = \sum_{i=1}^{n} m_i \, (N_v)_i$$

$\overline{N}_v = 0.66[Ni] + 1.71[Co] + 2.66[Fe] + 4.66[Cr+Mo+W] + 5.66[V]$
$+ 6.66[Zr] + 10.66[Nb]$ (all in mol%)

BY USING PHASE CALCULATION APPLYING ELECTRON THEORY (IN fcc ALLOY)

| Nv | | Nv | | Nv | |
|---|---|---|---|---|---|
| Co-20Cr-15W-0Fe | 2.58 | Co-20Cr-10W-0Fe | 2.50 | Co-20Cr-15W-10Ni (L605) | 2.47 |
| Co-20Cr-15W-5Fe | 2.63 | Co-20Cr-10W-5Fe | 2.58 | Co-29Cr-6Mo | 2.77 |
| Co-20Cr-15W-10Fe | 2.68 | Co-20Cr-10W-10Fe | 2.60 | Co-27Cr-5Mo | 2.68 |
| Co-20Cr-15W-15Fe | 2.74 | Co-20Cr-10W-15Fe | 2.65 | | |
| | | Co-20Cr-10W-20Fe | 2.70 | | |

(1) Co-20Cr-15W-5Fe  (2) Co-20Cr-15W-10Fe
(3) Co-20Cr-10W-5Fe  (4) Co-20Cr-10W-10Fe  (5) Co-20Cr-10W-15Fe

Fig. 9

Fig. 10

Fig. 11

Fig. 12

Fig. 13

Fig. 14

**Fig. 15**

Fig. 16

Fig. 17

STRUCTURES OBTAINED AFTER 1250°C-12h
HOMOGENIZATION THERMAL TREATMENT
Co-20Cr-10W-Fe

10W-0Fe

200 μm

10W-1Fe

200 μm

10W-3Fe

200 μm

10W-5Fe

200 μm

10W-10Fe

200 μm

10W-15Fe

200 μm

10W-20Fe

200 μm

# Fig. 18

STRUCTURES OBTAINED AFTER 1250°C-12h
HOMOGENIZATION THERMAL TREATMENT
Co-20Cr-15W-Fe

15W-0Fe

200 $\mu$ m

15W-1Fe

200 $\mu$ m

15W-3Fe

200 $\mu$ m

15W-5Fe

200 $\mu$ m

15W-10Fe

200 $\mu$ m

15W-15Fe

200 $\mu$ m

15W-20Fe

200 $\mu$ m

**Fig. 19**

Cr:20%, W:15%, Fe:5%

Fig. 20

Cr;20%, W;15%, Fe;10%

Fig. 21

Fig. 22

Cr : 20%, W : 10%, Fe : 5%

## Fig. 23

Fig. 24

Cr:20%, W:10%, Fe:15%

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007162121 A
- JP 2007517536 T
- WO 2011118615 A
- JP 2002130808 A

### Non-patent literature cited in the description

- *Acta Metall,* 1966, vol. 14, 853-865